# EUROPÄISCHE PATENTSCHRIFT

(11) **EP 0 726 296 B1**
(45) Veröffentlichungstag und Bekanntmachung des Hinweises auf die Patenterteilung: **16.08.2001**
(21) Anmeldenummer: 96810067.7
(22) Anmeldetag: 01.02.1996
(51) Int. Cl.: C09B 62/473, C07C 309/76

(54) **Azofarbstoffe**
Azo dyestuffs
Colorants azoiques

(30) Priorität: 10.02.1995 CH 40695
(43) Veröffentlichungstag der Anmeldung: 14.08.1996
(73) Patentinhaber: Ciba Specialty Chemicals Holding Inc., 4057 Basel (CH)
(72) Erfinder: Adam, Jean-Marie, Dr., F-68300 Rosenau (FR); Sutter, Peter, CH-4132 Muttenz (CH)

(56) Entgegenhaltungen:
- EP-A- 0 028 351
- FR-A- 1 427 724
- FR-A- 2 006 386
- GB-A- 984 802
- GB-A- 1 037 648
- US-A- 2 849 437
- US-A- 3 136 752

## Beschreibung

Die vorliegende Erfindung betrifft neue Azofarbstoffe, Verfahren zu ihrer Herstellung sowie ihre Verwendung zum Färben von Fasermaterialien aus wässrigem Bad oder zum Bedrucken von Fasermaterialien.

Aus der GB-A-984 802 und GB-A-1 037 648 sind Azofarbstoffe bekannt, welche einen spezifischen Acylaminorest enthalten und zum Färben und Bedrucken von Textilmaterialien, insbesondere Wolle verwendet werden.

EP-A-0 028 351 beschreibt Reaktivffarbstoffe enthaltend einen gegebenenfalls N-substituierten Sulfamoylrest und ihre Verwendung zum Färben hydroxylgruppenhaltiger und stickstoffhaltiger Materialien.

Aus der FR-A-1 427 724 sind Monoazofarbstoffe bekannt, welche einen Acylaminorest oder einen substituierten Chlortriazinaminorest enthalten und zum Färben von stickstoffhaltiger Materialien, insbesondere Wolle verwendet werden.

Gegenstand der vorliegenden Erfindung sind somit Verbindungen der Formel 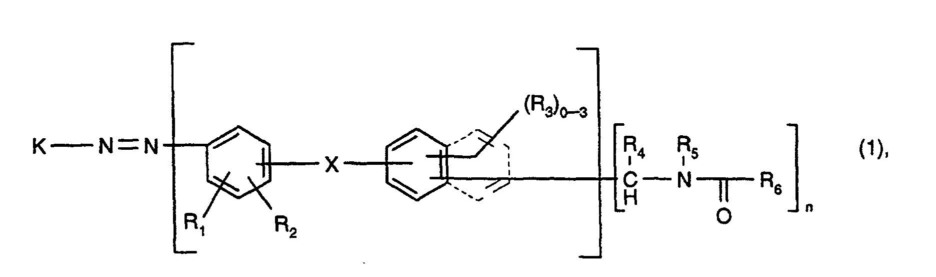 worin
K der Rest einer Acetessigsäureamid-Kupplungskomponente oder einer Kupplungskomponente der Benzol- oder Naphthalinreihe oder der heterocyclischen Reihe ist,
X ein Brückenglied der Formel -SO₂-O-, -SO₂-N(R₈)-, -COO- oder -CO-N(R₉)- bedeutet, wobei R₈ und R₉ unabhängig voneinander je Wasserstoff oder C₁-C₄-Alkyl sind,
R₁ und R₂ unabhängig voneinander je Wasserstoff, C₁-C₄-Alkyl, C₁-C₄-Alkoxy, Halogen oder Sulfo bedeuten,
(R₃)₀₋₃ für 0-3 gleiche oder verschiedene Reste R₃ aus der Gruppe C₁-C₄-Alkyl, C₁-C₄-Alkoxy, Halogen und Sulfo steht,
R₄ Wasserstoff, Carboxyl oder Trihalogenmethyl ist,
R₅ für Wasserstoff oder C₁-C₄-Alkyl steht,
R₆ C₁-C₄-Alkyl, ein Rest -CH₂Y oder -CHY-CH₂Y, worin Y Brom oder Chlor bedeutet, eine Gruppe -CH=CH₂ oder -CY₁=CH₂, worin Y₁ Chlor oder Brom bedeutet oder Phenyl ist,
   oder der Rest -N(R₅)-CO-R₆ einen Rest der Formel 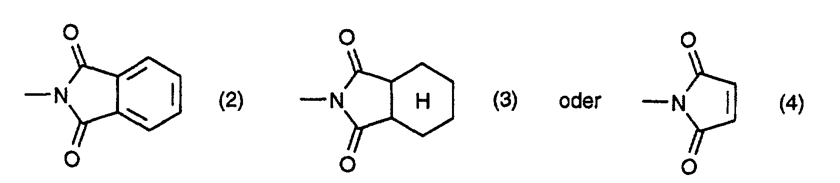 darstellt,
R₇ einen Rest -CHY-CH₂Y oder -CY=CH₂, worin Y Brom oder Chlor ist, bedeutet, und
n die Zahl 1, oder 2 bedeutet.

Die den Verbindungen der Formel (1) zugrunde liegenden Kupplungskomponenten K-H sind an sich bekannt und in grosser Zahl beschrieben z.B. in Venkataraman "The Chemistry of Synthetic Dyes" Band 6, Seiten 213-297, Academic Press, New York, London 1972.

K steht bevorzugt für den Rest einer Benzol-, Naphthalin-, Pyrazolon-, Aminopyrazol-, Pyridon-, Pyrimidin-, Indol-, Naphthylimidazol-, Diphenylamin-, Pyrazolo[2,3-a]pyrimidin-, Tetrahydrochinolin- oder Acetessigsäureamid-Kupplungskomponente, wobei die genannten Reste weitersubstituiert sein können.

Geeignete Substituenten am Rest K sind z.B.: C₁-C₆-Alkyl, worunter generell Methyl, Ethyl, n- oder iso-Propyl, n-, iso-, sec.- oder tert.-Butyl oder geradkettiges oder verzweigtes Pentyl oder Hexyl zu verstehen ist; C₁-C₄-Alkoxy, worunter generell Methoxy, Ethoxy, n- oder iso-Propoxy oder n-, iso-, sec.- oder tert.-Butoxy zu verstehen ist; Phenoxy; gegebenenfalls durch Hydroxy substituiertes C₂-C₆-Alkanoylamino, z.B. Acetylamino, Hydroxyacetylamino oder Propionylamino; Benzoylamino; Amino; gegebenenfalls im Alkylteil z.B. durch Hydroxy, C₁-C₄-Alkoxy, Carboxy, Cyano, Halogen, Sulfo, Sulfato, Phenyl oder Sulfophenyl substituiertes N-C₁-C₄-Alkyl- oder N,N-Di-C₁-C₄-Alkylamino, z.B. Methylamino, Ethylamino, N,N-Dimethylamino, N,N-Diethylamino, β-Cyanoethylamino, β-Hydroxyethylamino, N,N-Di-β-Hydroxyethylamino, β-Sulfoethylamino, γ-Sulfo-n-propylamino, β-Sulfoethylamino, N-Ethyl-N-(3-Sulfobenzyl)-amino, N-(β-Sulfoethyl)-N-benzylamino; Cyclohexylamino; gegebenenfalls im Phenylteil durch C₁-C₄-Alkyl, C₁-C₄-Alkoxy, Halogen oder Sulfo substituiertes N-Phenylamino oder N-C₁-C₄-Alkyl-N-phenylamino; C₂-C₄-Alkoxycarbonyl, z.B. Methoxy- oder Ethoxycarbonyl; Trifluoromethyl; Nitro; Cyano; Halogen, worunter generell z.B. Fluor, Brom oder insbesondere Chlor zu verstehen ist; Ureido; Hydroxy; Carboxy; Sulfo; Sulfomethyl; Carbamoyl; Sulfamoyl; gegebenenfalls im Phenylteil durch Sulfo oder Carboxy substituiertes N-Phenylsulfamoyl oder N-C₁-C₄-Alkyl-N-phenylsulfamoyl; Methyl- oder Ethylsulfonyl; oder gegebenenfalls z.B. durch C₁-C₄-Alkyl, C₁-C₄-Alkoxy, Halogen, Sulfo, Amino, N-C₁-C₄-Alkyl- oder N,N-Di-C₁-C₄-Alkylamino oder Phenylamino substituiertes Phenylazo oder Naphthylazo.

Bevorzugte Bedeutungen von K sind: Ein Phenyl- oder Naphthylrest, der einen oder mehrere Substituenten aus der Gruppe Sulfo, Hydroxy, C₁-C₄-Alkoxy, Amino, N-C₁-C₄-Alkyl- oder N,N-Di-C₁-C₄-Alkylamino, Acetylamino, Benzoylamino, C₁-C₄-Alkyl, und unsubstituiertes oder durch C₁-C₄-Alkyl, C₁-C₄-Alkoxy, Halogen, Sulfo, Amino, N-C₁-C₄-Alkyl- oder N,N-Di-C₁-C₄-Alkylamino oder Phenylamino substituiertes Phenylazo oder Naphthylazo trägt; ein unsubstituierter oder durch C₁-C₄-Alkyl, C₁-C₄-Alkoxy, Sulfo oder Halogen substituierter 1-Phenylpyrazol-5-on- oder 1-Phenyl-5-aminopyrazolrest; ein unsubstituierter oder durch C₁-C₄-Alkyl, C₁-C₄-Alkoxy, Sulfo oder Halogen substituierter Indolrest; und ein unsubstituierter oder durch C₁-C₆-Alkyl, Sulfo, Hydroxy oder Phenylamino, welches seinerseits durch C₁-C₄-Alkyl, C₁-C₄-Alkoxy, Sulfo oder Halogen substituiert sein kann, substituierter Naphthylimidazolrest.

K steht besonders bevorzugt für einen 1- oder 2-Naphthylrest, der einen oder mehrere Substituenten aus der Gruppe Hydroxy, Amino, Acetylamino, Sulfo und Chlor trägt, oder für einen 1-Phenyl-pyrazol-5-on- oder 1-Phenyl-5-aminopyrazolrest, der jeweils einen oder mehrere Substituenten aus der Gruppe Methyl, Methoxy, Sulfo und Chlor trägt.

R₁ und R₂ bedeuten unabhängig voneinander je bevorzugt Wasserstoff, Methyl, Ethyl, Methoxy, Ethoxy, Chlor oder Sulfo und besonders bevorzugt Wasserstoff, Methyl, Methoxy, Chlor oder Sulfo. Eine bevorzugte Ausführungsform der Erfindung betrifft Verbindungen der Formel (1), worin R₁ Wasserstoff und R₂ Wasserstoff, Methyl, Methoxy, Chlor oder Sulfo bedeuten.

(R₃)₀₋₃ steht bevorzugt für 0 bis 3 gleiche oder verschiedene Reste aus der Gruppe Methyl, Ethyl, Methoxy, Ethoxy, Chlor oder Sulfo und besonders bevorzugt aus der Gruppe Methyl, Methoxy, Chlor oder Sulfo. Eine bevorzugte Ausführungsform der Erfindung betrifft Verbindungen der Formel (1), worin (R₃)₀₋₃ für 0 bis 3 Methylreste steht.

Die unter der Formel (1) genannten Brückenglieder X sind so zu verstehen, dass ihr S- bzw. C-Atom jeweils an den linken die Reste R₁ und R₂ tragenden Phenylrest und das O- bzw. N-Atom an den rechten die Gruppe (R₃)₀₋₃ tragenden Phenyl- oder Naphthylrest gebunden sind. X steht bevorzugt für ein Brückenglied der Formel -SO₂-O-, -SO₂-N(C₁-C₄-Alkyl)-, -COO- oder -CO-N(C₁-C₄-Alkyl)- und besonders bevorzugt für die Gruppe -SO₂-O-.

R₄ bedeutet bevorzugt Wasserstoff, Carboxyl oder Trifluormethyl und besonders bevorzugt Wasserstoff.

R₅ steht bevorzugt für Wasserstoff, Methyl oder Ethyl und besonders bevorzugt für Wasserstoff.

R₆ steht bevorzugt für einen Rest -CH₂Cl, -CHBr-CH₂Br oder CBr=CH₂.

R₇ bedeutet bevorzugt den Rest -CHBr-CH₂Br oder -CBr=CH₂.

Die Verbindungen der Formel (1) weisen eine oder mehrere Sulfogruppen auf, wobei diese entweder in Form ihrer freien Sulfosäure oder vorzugsweise als deren Salz vorliegen. Als Salze kommen z.B. die Alkali- oder Erdalkalisalze, das Ammoniumsalz oder Salze organischer Amine in Betracht. Als Beispiele seien genannt die Natrium-, Kalium-, Lithium- oder Ammoniumsalze, die Salze des Mono-, Di- oder Triethanolamins oder Mischungen davon. Die bevorzugten Verbindungen der Formel (1) weisen eine Sulfogruppe auf.

Die Verbindungen der Formel (1) können hergestellt werden, z.B. indem man ein Amin der Formel 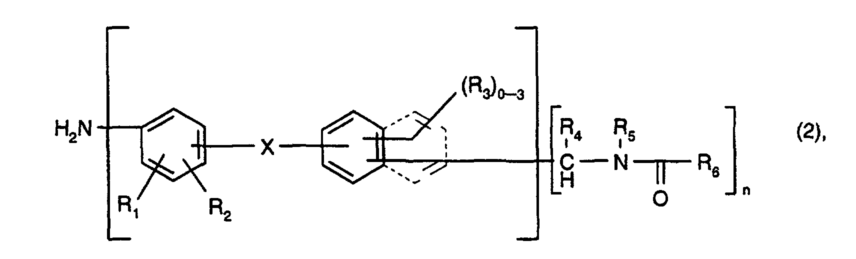 worin R₁, R₂, (R₃)₀₋₃, R₄, R₅, R₆, R₇, X, und n jeweils die zuvor angegebene Bedeutung haben, diazotiert und mit einer Kupplungskomponente der Formel

K-H (3),

worin K die zuvor angegebene Bedeutung hat, kuppelt. Die Diazotierung der Amine der Formel (2) kann dabei in üblicher Weise, z.B. mit Nitriten wie z.B. Natriumnitrit in einem sauren, z.B. salzsauren, Medium bei Temperaturen von z.B. 0-15°C, erfolgen. Die Kupplung der diazotierten Amine der Formel (2) mit der Kupplungskomponente der Formel (3) erfolgt vorteilhaft z.B. in einem wässrigen oder wässrig-organischen Medium bei Temperaturen von z.B. 0-30°C und einem neutralen oder leicht sauren pH-Wert.

Die Verbindungen der Formel (2) sind neu und stellen einen weiteren Gegenstand der Erfindung dar. Sie können erhalten werden, z.B. indem man eine Verbindung der Formel 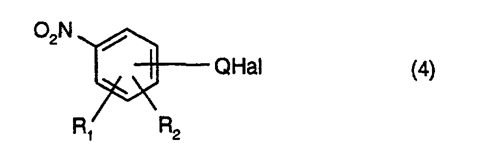 oder eine entsprechende Verbindung, mit einer Verbindung der Formel 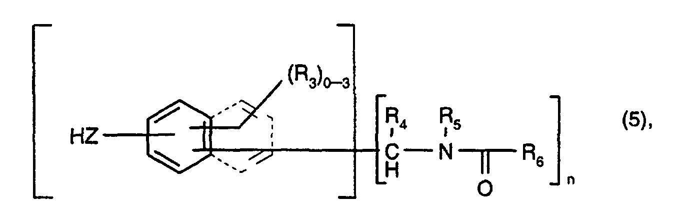 oder einer entsprechenden Verbindung, die eine Vorstufe des Rests -(CHR₄-NR₅-COR₆)ₙ enthält, umsetzt, die erhaltene Nitroverbindung anschliessend z.B. durch katalytische Hydrierung in das entsprechende Amin umwandelt, und im Fall der Verwendung von Vorstufen der Verbindungen der Formeln (4) und/oder (5) diese in die Endverbindungen umwandelt, worin R₁, R₂, (R₃)₀₋₃, R₄, R₅, R₆, R₇ und n jeweils die zuvor angegebene Bedeutung haben, -QHal z.B. der Rest-COCl oder -SO₂Cl ist, und -ZH einen Rest -OH oder -NH(R₈), worin R₈ die zuvor angegebene Bedeutung hat, bedeutet.

Die Reaktion der Verbindungen der Formeln (4) und (5) kann in allgemein üblicher Weise, z.B. in einem wässrigem oder organischen Medium in Gegenwart einer Base bei erhöhter Temperatur, durchgeführt werden. Auch die katalytische Hydrierung kann unter üblichen Bedingungen, z.B. mit einem Pd/C-Katalysator oder Raney-Nickel in einem organischen Lösungsmittel, z.B. in Tetrahydrofuran oder Dimethylformamid, durchgeführt werden. Setzt man in das Verfahren anstelle der Verbindung der Formel (4) eine geeignete Vorstufe ein, so kann dies z.B. eine Verbindung sein, die anstelle der Gruppe -(NHCOR₇)ₘ₁ einen in eine Aminogruppe überführbaren Substituenten, z.B. Chlor oder Nitro, aufweist, der nach seiner Ueberführung in die Aminogruppe z.B.mit einer Verbindung Cl-COR₇ umgesetzt werden kann. Setzt man in das Verfahren anstelle der Verbindung der Formel (5) eine geeignete Vorstufe ein, so kann dies z.B. eine Verbindung sein, die anstelle der Gruppe -(CHR₄-NR₅-COR₆)ₙ eine Gruppe -(CH₂R₄)ₙ aufweist, die anschliessend z.B. mittels Einhom-Reaktion in die Endverbindung umgewandelt werden kann.

Die Verbindungen der Formeln (4) und (5) bzw. entsprechende Vorstufen davon sind bekannt oder können nach bekannten Methoden hergestellt werden.

Die erfindungsgemässen Verbindungen der Formel (1) eignen sich als Farbstoffe zum Färben oder Bedrucken der verschiedensten Fasermaterialien, z.B. von hydroxylgruppenhaltigen oder stickstoffhaltigen Fasermaterialien. Solche Fasermaterialien sind z.B. die natürlichen Cellulosefasern, wie Baumwolle, Leinen, Jute oder Hanf, sowie modifizierte Cellulosefasern wie Zellstoff oder regenerierte Cellulose. Insbesondere eignen sich die Verbindungen der Formel (1) als Farbstoffe zum Färben oder Bedrucken von natürlichen Polyamidfasermaterialien, z.B. Seide oder Wolle, synthetischen Polyamidfasermaterialien, z.B. Polyamid 6 oder Polyamid 6.6, oder von Woll- und synthetischen Polyamidmischgeweben. Die Verbindungen der Formel (1) sind besonders geeignet als Farbstoffe zum Färben oder Bedrucken von natürlichen Polyamidfasermaterialien, insbesondere von Wolle.

Das genannte Textilfasermaterial kann dabei in den verschiedensten Verarbeitungsformen vorliegen, wie z.B. als Faser, Garn, Flocke, Gewebe oder Gewirke.

Die erfindungsgemässen Farbstoffe der Formel (1) eignen sich für die üblichen Färbe- und Druckverfahren und lassen sich auf verschiedenste Weise auf das Fasermaterial applizieren und fixieren, insbesondere in Form von wässrigen Farbstofflösungen oder -druckpasten. Sie eignen sich sowohl für das Ausziehverfahren als auch zum Foulard-Färben, wonach die Ware mit wässrigen, gegebenenfalls salzhaltigen Farbstofflösungen imprägniert wird, und die Farbstoffe nach einer Alkalibehandlung oder in Gegenwart von Alkali, gegebenenfalls unter Wärmeeinwirkung, fixiert werden. Die erfindungsgemässen Farbstoffe der Formel (1) sind ebenfalls für das sogenannte Kaltverweilverfahren geeignet, wonach der Farbstoff zusammen mit dem Alkali auf dem Foulard aufgebracht wird und danach durch mehrstündiges Lagern bei Raumtemperatur fixiert wird.

Das Färben von natürlichen und synthetischen Polyamidfasermaterialien, insbesondere von Wolle, erfolgt vorzugsweise nach dem Ausziehverfahren, bei einem pH-Wert von ca. 3 bis 7, insbesondere 3 bis 5, und bei Temperaturen von z.B. 70 bis 110°C und insbesondere 90 bis 100°C.

Die Färbeflotten oder Druckpasten können ausser Wasser und den Farbstoffen weitere Zusätze, z.B. Salze, Puffersubstanzen, Netzmittel, Antischaummittel, Egalisiermittel oder die Eigenschaften des Textilmaterials beeinflussende Mittel, z.B. Weichmachungsmittel, Zusätze zum Flammfestausrüsten oder schmutz-, wasser- und öl-abweisende Mittel sowie wasserenthärtende Mittel und natürliche oder synthetische Verdicker, z.B. Alginate oder Celluloseether, enthalten.

Die erfindungsgemässen Farbstoffe der Formel (1) ergeben egale Färbungen und Drucke mit guten Allgemeinechtheiten, insbesondere guter Wasch-, Reib-, Nass-, Nassreib- und Lichtechtheit. Die Farbstoffe der Formel (1) zeichnen sich ferner durch gleichmässigen Farbaufbau, gutes Aufziehverhalten, hohe Fixiergrade sowie durch gute Kombinierbarkeit mit anderen Farbstoffen aus. Weiterhin kann bei den erfindungsgemässen Farbstoffen der Formel (1) auf die sonst übliche Nachbehandlung der Färbungen und Drucke mit sogenannten Fixiermitteln verzichtet werden.

In den folgenden Beispielen stehen Teile für Gewichtsteile. Die Temperaturen sind Celsiusgrade. Die Beziehung zwischen Gewichtsteilen und Volumenteilen ist dieselbe wie diejenige zwischen Gramm und Kubikzentimeter.

Beispiel 1: 108 Teile p-Kresol und 250 Teile N-Hydroxymethyl-chloracetamid werden bei 0-20°C in 500 Teile 98%ige Schwefelsäure eingetragen. Man lässt über Nacht ausreagieren, trägt dann das Reaktionsgemisch auf 1500 Teile Eis aus und filtriert die Verbindung der Formel 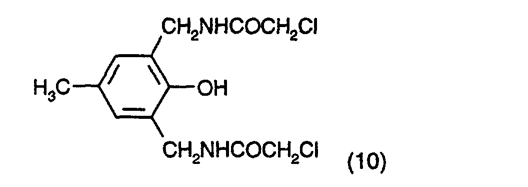 ab, welche anschliessend in üblicher Weise gewaschen und getrocknet wird.

19,2 Teile der Verbindung der Formel (10) werden in 250 Teilen Aceton gelöst und mit 6,1 Teilen Triethylamin versetzt. Man tropft innerhalb von 5 Minuten eine Lösung von 13,3 Teilen 2-Nitrobenzol-1-sulfochlorid in 50 Teilen Aceton zu, erhitzt das Ganze 15 Minuten unter Rückfluss und dampft zur Trockne ein. Der Rückstand wird in 300 Teilen Wasser verrührt und der Feststoff abfiltriert. Der Feststoff wird anschliessend in ca. 300 Teilen Methanol aufgekocht, erneut abfiltriert und getrocknet. Man erhält 24 Teile der Verbindung der Formel

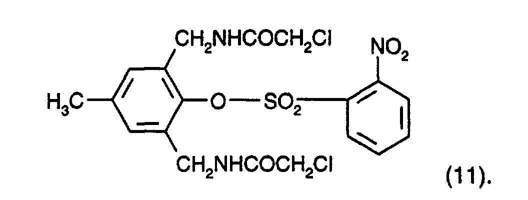

5,1 Teile der Verbindung der Formel (11) werden katalytisch mit 0,5 Teilen 5% Pd/C hydriert. Nach dem Eindampfen erhält man 4,4 Teile der Verbindung der Formel

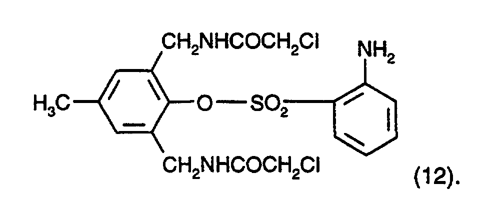

9,5 Teile der Verbindung der Formel (12) werden in 45 Teilen Eisessig, 11 Teilen konz. Hcl und 11 Teilen Wasser gelöst und unter Zugabe von 5,1 Teilen 4N Natriumnitritlösung bei 0-5°C diazotiert. Danach werden 10,5 Teile Natriumacetat addiert (pH 2,5) und im Anschluss daran 5,3 Teile 5-Amino-3-methyl-1-(3-sulfophenyl)-pyrazol bei 0-5°C zugegeben. Man lässt 3 Stunden bei Raumtemperatur rühren und trägt nach einer weiteren Stunde auf 120 Teile 25%ige Kochsalzlösung aus. Die flüssige Phase wird abdekantiert und der Rückstand in 200 Teilen Wasser gelöst. Man stellt mit Natriumhydroxidlösung auf pH 7,5 und salzt den Farbstoff mit Kochsalz aus. Nach dem Abfiltrieren und Trocknen erhält man den Farbstoff der Formel 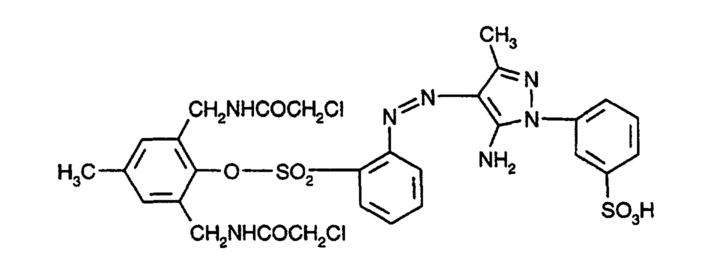 der Wolle in einer gelben Nuance mit guten Allgemeinechtheiten färbt.

Analog wie in Beispiel 1 beschrieben lassen sich die folgenden Farbstoffe herstellen. 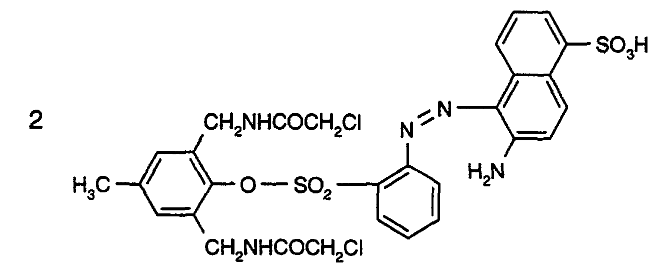 und

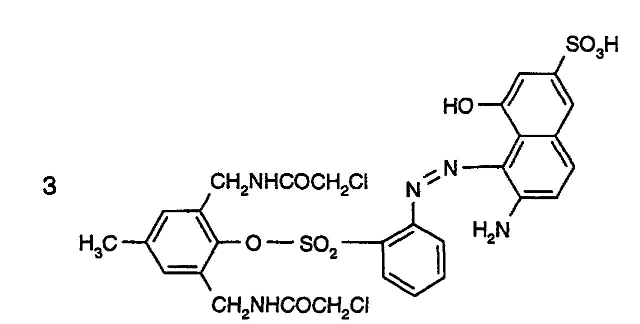

Beispiele 4-6: 139 Teile Mesitol werden in 1200 Teilen Aceton gelöst und 117 Teile Triethylamin zugegeben. Man tropft innerhalb von 20 Minuten eine Lösung von 267 Teilen 2-Nitrobenzol-1 -sulfochlorid in 600 Teilen Aceton zu, erhitzt das Ganze 1 Stunde unter Rückfluss und dampft zur Trockne ein. Der Rückstand wird in Wasser verrührt und der Feststoff abfiltriert. Der Feststoff wird anschliessend heiss in ca. 700 Teilen Aceton gelöst, dann 500 Teile Aceton abdestilliert und 500 Teile Methanol addiert. Der ausgefallene Feststoff wird erneut abfiltriert und getrocknet. Man erhält 209 Teile der Verbindung der Formel

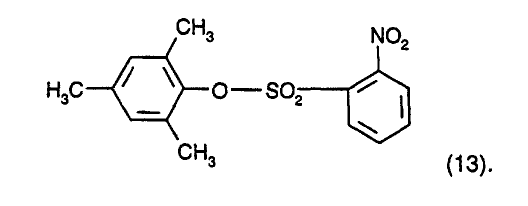

161 Teile der Verbindung der Formel (13) werden bei erhöhter Temperatur (60°C) unter Druck (40 bar) mit Raney-Nickel katalytisch hydriert. Nach dem Eindampfen erhält man 142 Teile der Verbindung der Formel

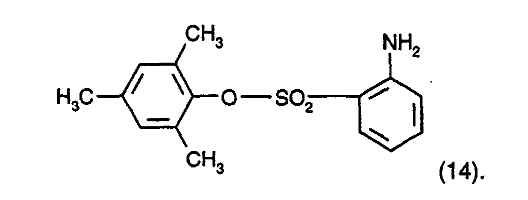

58,3 Teile der Verbindung der Formel (14) und 50,4 Teile N-Hydroxymethyl-chloracetamid werden homogenisiert und bei 0-5°C zu 110 Teilen 98%ige Schwefelsäure gegeben. Nach 4 Stunden trägt man auf 1000 Teile Eis aus, filtriert den Niederschlag ab und wäscht ihn neutral. Nach dem Trocknen ergeben sich 81 Teile der Verbindung der Formel

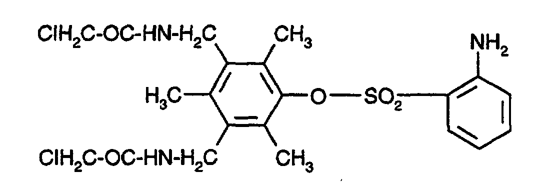

Analog wie im Beispiel 1 beschrieben, werden daraus die Farbstoffe der Formeln 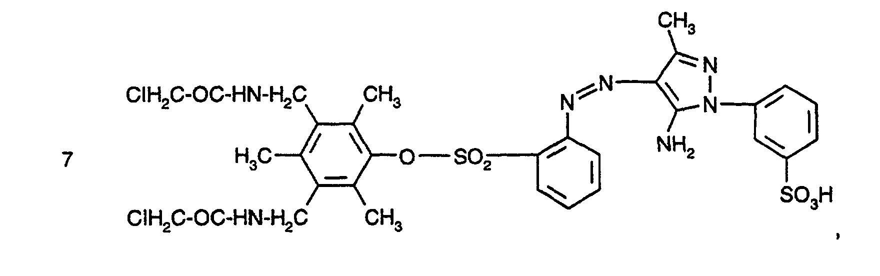 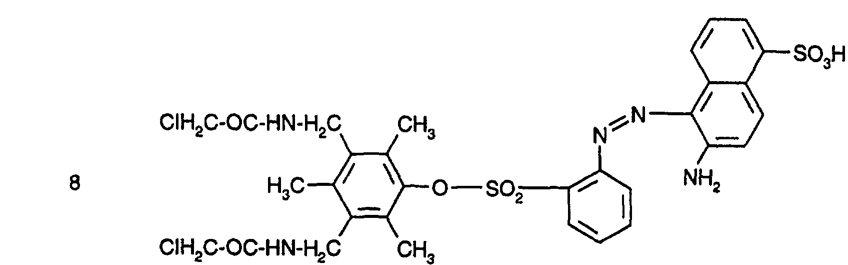 und 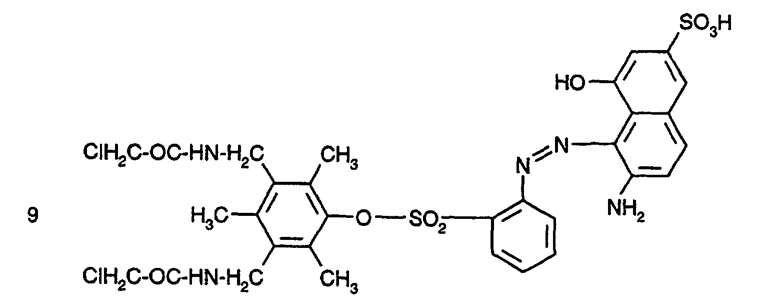 hergestellt.

## Patentansprüche

1. Verbindungen der Formel 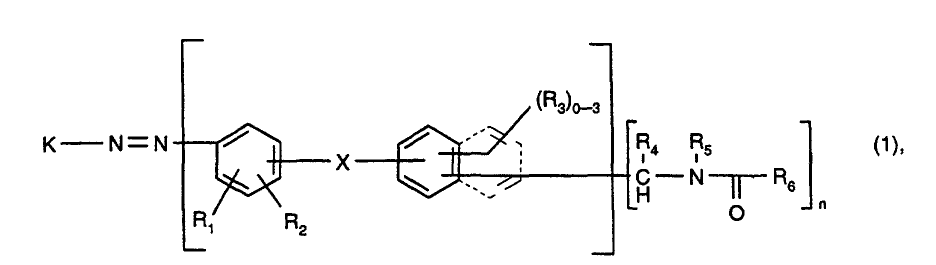 worin
K der Rest einer Acetessigsäureamld-Kupplungskomponente oder einer Kupplungskomponente der Benzol- oder Naphthalinreihe oder der heterocyclischen Reihe ist,
X ein Brückenglied der Formel -SO₂-O-, -SO₂-N(R₈)-, -COO- oder -CO-N(R₉)- bedeutet, wobei R₈ und R₉ unabhängig voneinander je Wasserstoff oder C₁-C₄-Alkyl sind,
R₁ und R₂ unabhängig voneinander je Wasserstoff, C₁-C₄-Alkyl, C₁-C₄-Alkoxy, Halogen oder Sulfo bedeuten,
(R₃)₀₋₃ für 0-3 gleiche oder verschiedene Reste R₃ aus der Gruppe C₁-C₄-Alkyl, C₁-C₄-Alkoxy, Halogen und Sulfo steht,
R₄ Wasserstoff, Carboxyl oder Trihalogenmethyl ist,
R₅ für Wasserstoff oder C₁-C₄-Alkyl steht,
R₆ C₁-C₄-Alkyl, ein Rest -CH₂Y oder -CHY-CH₂Y, worin Y Brom oder Chlor bedeutet, eine Gruppe -CH=CH₂ oder -CY₁=CH₂, worin Y₁ Chlor oder Brom bedeutet oder Phenyl ist, oder der Rest -N(R₅)-CO-R₆ einen Rest der Formel 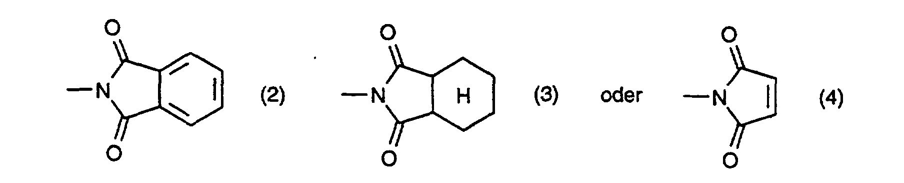 darstellt,
R₇ einen Rest -CHY-CH₂Y oder -CY=CH₂, worin Y Brom oder Chlor ist, bedeutet, und
n die Zahl 1, oder 2 bedeutet.

2. Verbindungen gemäss Anspruch 1, dadurch gekennzeichnet, dass K für den Rest einer Benzol-, Naphthalin-, Pyrazolon-, Aminopyrazol-, Pyridon-, Pyrimidin-, Indol-, Naphthylimidazol-, Diphenylamin-, Pyrazolo[2,3-a]pyrimidin-, Tetrahydrochinolin- oder Acetessigsäureamid-Kupplungskomponente steht.

3. Verbindungen gemäss Anspruch 1 oder 2, dadurch gekennzeichnet, dass K ein Phenyl- oder Naphthylrest, der einen oder mehrere Substituenten aus der Gruppe Sulfo, Hydroxy, C₁-C₄-Alkoxy, Amino, N-C₁-C₄-Alkyl- oder N,N-Di-C₁-C₄-Alkylamino, Acetylamino, Benzoylamino, C₁-C₄-Alkyl, und unsubstituiertes oder durch C₁-C₄-Alkyl, C₁-C₄-Alkoxy, Halogen, Sulfo, Amino, N-C₁-C₄-Alkyl- oder N,N-Di-C₁-C₄-Alkylamino oder Phenylamino substituiertes Phenylazo oder Naphthylazo trägt; ein unsubstituierter oder durch C₁-C₄-Alkyl, C₁-C₄-Alkoxy, Sulfo oder Halogen substituierter 1-Phenylpyrazol-5-on- oder 1-Phenyl-5-aminopyrazolrest; ein unsubstituierter oder durch C₁-C₄-Alkyl, C₁-C₄-Alkoxy, Sulfo oder Halogen substituierter Indolrest; oder ein unsubstituierter oder durch C₁-C₆-Alkyl, Sulfo, Hydroxy oder Phenylamino, welches seinerseits durch C₁-C₄-Alkyl, C₁-C₄-Alkoxy, Sulfo oder Halogen substituiert sein kann, substituierter Naphthylimidazolrest ist.

4. Verbindungen gemäss einem der Ansprüche 1 bis 3, dadurch gekennzeichnet, dass K für einen 1- oder 2-Naphthylrest, der einen oder mehrere Substituenten aus der Gruppe Hydroxy, Amino, Acetylamino, Sulfo und Chlor trägt, oder für einen 1-Phenyl-pyrazol-5-on- oder 1-Phenyl-5-aminopyrazolrest, der jeweils einen oder mehrere Substituenten aus der Gruppe Methyl, Methoxy, Sulfo und Chlor trägt, steht.

5. Verbindungen gemäss einem der Ansprüche 1 bis 4, dadurch gekennzeichnet, dass R₁ und R₂ unabhängig voneinander je Wasserstoff, Methyl, Ethyl, Methoxy, Ethoxy, Chlor oder Sulfo bedeuten.

6. Verbindungen gemäss einem der Ansprüche 1 bis 5, dadurch gekennzeichnet, dass (R₃)₀₋₃ für 0 bis 3 gleiche oder verschiedene Reste aus der Gruppe Methyl, Ethyl, Methoxy, Ethoxy, Chlor und Sulfo steht.

7. Verbindungen gemäss einem der Ansprüche 1 bis 6, dadurch gekennzeichnet, dass X ein Brückenglied der Formel -SO₂-O-, -SO₂-N(C₁-C₄-Alkyl)-, -COO- oder -CO-N(C₁-C₄-Alkyl)- und vorzugsweise der Formel -SO₂-O- bedeutet.

8. Verbindungen gemäss Anspruch 1, dadurch gekennzeichnet, dass R₆ den Rest -CH₂Cl, -CHBr-CH₂Br oder -CBr=CH₂ bedeutet.

9. Verbindungen gemäss einem der Ansprüche 1 bis 8, dadurch gekennzeichnet, dass sie eine oder mehrere Sulfogruppen und vorzugsweise eine Sulfogruppe aufweisen.

10. Verfahren zur Herstellung von Verbindungen der Formel (1) gemäss Anspruch 1, dadurch gekennzeichnet, dass man ein Amin der Formel 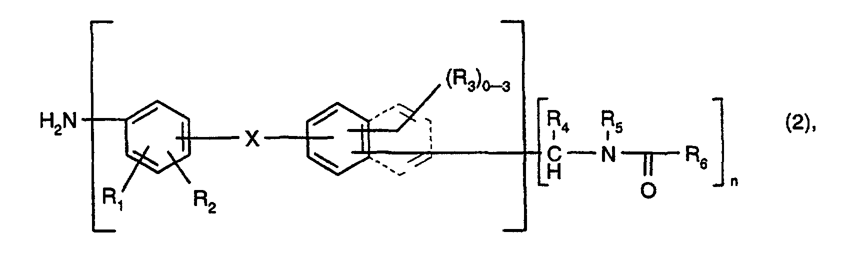 worin R₁, R₂, (R₃)₀₋₃, R₄, R₅, R₆, R₇, X, und n jeweils die im Anspruch 1 angegebene Bedeutung haben, diazotiert und mit einer Kupplungskomponente der Formel
K-H (3),
worin K die im Anspruch 1 angegebene Bedeutung hat, kuppelt.

11. Verbindungen der Formel (2) gemäss Anspruch 10.

12. Verwendung von Verbindungen der Formel (1) gemäss einem der Ansprüche 1 bis 9 zum Färben oder Bedrucken von stickstoffhaltigen oder hydroxylgruppenhaltigen Fasermaterialien.

13. Verwendung gemäss Anspruch 12 zum Färben oder Bedrucken von natürlichen oder synthetischen Polyamidfasermaterialien, insbesondere Wolle.

## Claims

1. A compound of formula 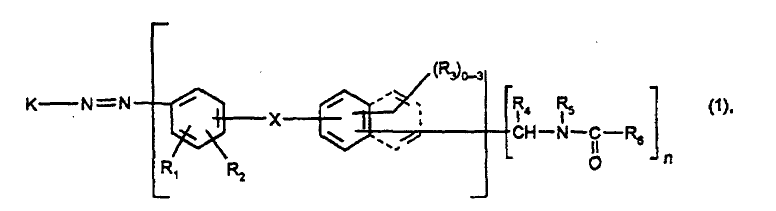 in which
K is the radical of an acetoacetamide coupling component or of a coupling component of the benzene or naphthalene series or of the heterocyclic series,
X is a bridge member of formula -SO₂-O-, -SO₂-N(R₈)-, -COO- or -CO-N(R₉)-, where R₈ and R₉ are each independently of the other hydrogen or C₁-C₄alkyl,
R₁ and R₂ are each independently of the other hydrogen, C₁-C₄alkyl, C₁-C₄alkoxy, halogen or sulfo,
(R₃)₀₋₃ is 0-3 identical or different radicals R3 selected from the group consisting of C₁-C₄alkyl, C₁-C₄alkoxy, halogen and sulfo,
R₄ is hydrogen, carboxyl or trihalomethyl,
R₅ is hydrogen or C₁-C₄alkyl,
R₆ is C₁-C₄alkyl, a -CH₂Y or -CHY-CH₂Y radical in which Y is bromo or chloro, a group -CH=CH₂ or -CY₁=CH₂ in which Y₁ is chloro or bromo, or R₆ is phenyl,
or the -N(R₅)-CO-R₆ radical is a radical of formula 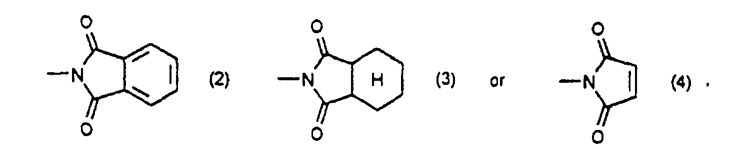
R₇ is a -CHY-CH₂Y or -CY=CH₂ radical in which Y is bromo or chloro, and
N is 1 or 2.

2. A compound according to claim 1, wherein K is the radical of a benzene, naphthalene, pyrazolone, aminopyrazole, pyridone, pyrimidine, indole, naphthylimidazole, diphenylamine, pyrazolo [2,3a]pyrimidine, tetrahydroquinoline or acetoaceamide coupling component.

3. A compound according to either claim 1 or claim 2, wherein K is a phenyl or naphthyl radical carrying one or more than one substituent selected from the group consisting of sulfo, hydroxy, C₁-C₄alkoxy, amino, N-C₁-C₄alkylamino or N,N-di-C₁-C₄alkylamino, acetylamino, benzoylamino, C₁-C₄alkyl; and phenylazo or naphthylazo, each of which is unsubstituted or substituted by C₁-C₄alkyl; C₁-C₄alkoxy, halogen, sulfo, amino, N-C₁-C₄alkylamino, N,N-di-C₁-C₄alkylamino or phenylamino; a 1-phenylpyrazol-5-one or 1-phenyl-5-aminopyrazole radical, each of which is unsubstituted or substituted by C₁-C₄alkyl, C₁-C₄alkoxy, sulfo or halogen; an indole radical which is unsubstituted or substituted by C₁-C₄alkyl, C₁-C₄alkoxy, sulfo or halogen; or a naphthylimidazole radical which is unsubstituted or substituted by C₁-C₆alkyl, sulfo, hydroxy or phenylamino, which phenylamino can in turn be substituted by C₁-C₄alkyl, C₁-C₄alkoxy, sulfo or halogen.

4. A compound according to any one of claims 1 to 3, wherein K is a 1- or 2-naphthyl radical carrying one or more than one substituent selected from the group consisting of hydroxy, amino, acetylamino, sulfo and chloro, or a 1-phenylpyrazol-5-one or 1-phenyl-5-aminopyrazole radical, each of which carries one or more than one substituent selected from the group consisting of methyl, methoxy, sulfo and chloro.

5. A compound according to any one of claims 1 to 4, wherein R₁ and R₂ are each independently of the other hydrogen, methyl, ethyl, methoxy, ethoxy, chloro or sulfo.

6. A compound according to any one of claims 1 to 5, wherein (R₃)₀₋₃ is 0 to 3 identical or different radicals selected from the group consisting of methyl, ethyl, methoxy, ethoxy, chloro and sulfo.

7. A compound according to any one of claims 1 to 6, wherein X is a bridge member of formula -SO₂-O-, -SO₂-N(C₁-C₄alkyl)-, -COO- or -CO-N(C₁-C₄alkyl)- and, preferably, of formula -SO₂-O-.

8. A compound according to claim 1, wherein R₆ is the -CH₂Cl, -CHBr-CH₂Br or -CBr=CH₂ radical.

9. A compound according to any one of claims 1 to 8, which compound contains one or more than one sulfo group and, preferably, one sulfo group.

10. A process for the preparation of a compound of formula (1) according to claim 1, which comprises diazotising an amine of formula 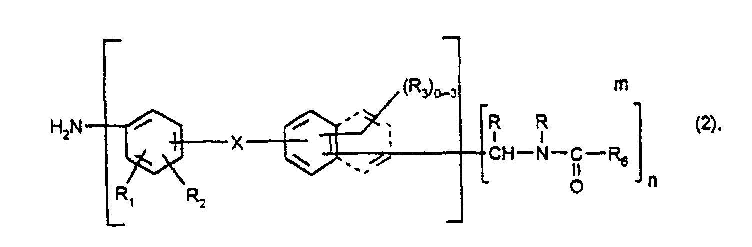 in which R₁, R₂, (R₃)₀₋₃, R₄, R₅, R₆, R₇, X, and n each have the meaning specified in claim 1, and coupling the diazonium compound so obtained with a coupling component of formula
K-H (3),
in which K is as specified in claim 1.

11. A compound of formula (2) according to claim 10.

12. Use of a compound of formula (1) according to any one of claims 1 to 9 for dyeing or printing nitrogen-containing or hydroxyl group containing fibre materials.

13. Use according to claim 12 for dyeing or printing natural or synthetic polyamide fibre materials, especially wool.

## Revendications

1. Composés de formule 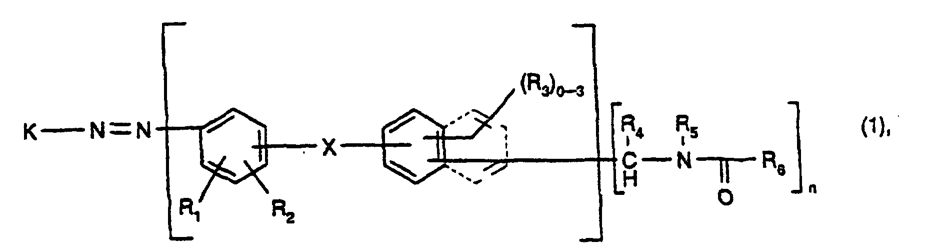 où
K représente le reste d'un composant de copulation du type acétamido ou d'un composant de copulation de la série du benzène ou du naphtalène ou de la série hétérocyclique,
X représente un élément de pontage de formule -SO₂-O-, -SO₂-N(R₈)-, -COO- ou -CO-N(R₉)-, où R₈ et R₉ représentent chacun, indépendamment l'un de l'autre, un atome d'hydrogène ou un groupe alkyle en C₁-C₄,
R₁ et R₂ représentent chacun, indépendamment l'un de l'autre, un atome d'hydrogène, des groupes alkyle en C₁-C₄, alkoxy en C₁-C₄, halogène ou sulfo,
(R₃)₀₋₃ représente 0 à 3 restes R₃ identiques ou différents pris parmi les restes alkyle en C₁-C₄, alkoxy en C₁-C₄, halogène et sulfo,
R₄ représente un atome d'hydrogène, des groupes carboxyle ou trihalogénométhyle,
R₅ représente un atome d'hydrogène ou un groupe alkyle en C₁-C₄,
R₆ représente un groupe alkyle en C₁-C₄, un reste -CH₂-Y ou -CHY-CH₂Y, où Y représente un atome de brome ou de chlore, un groupe -CH=CH₂ ou -CY₁=CH₂, où Y₁ représente
un atome de chlore ou de brome ou représente un groupe phényle,
ou le reste -N(R₅)-CO-R₆ représente un reste de formule 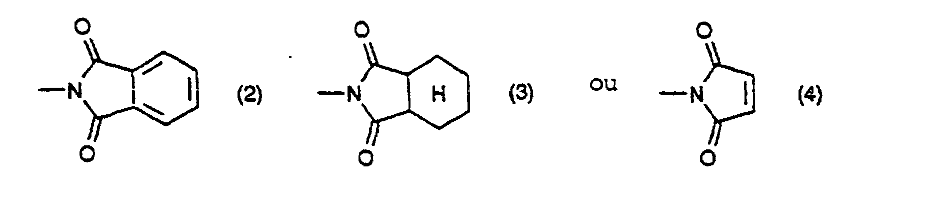
R₇ représente un reste -CHY-CH₂Y ou -CH=CH₂, où Y représente un atome de brome ou de chlore, et
n vaut 1 ou 2.

2. Composés selon la revendication 1, caractérisés en ce que K représente le reste d'un composant de copulation du type benzène, naphtalène, pyrazolone, aminopyrazole, pyridone, pyrimidine, indole, naphtylimidazole, diphénylamine, pyrazolo-[2,3-a]-pyrimidine, tétrahydroquinoléine ou acétamido.

3. Composés selon la revendication 1 ou 2, caractérisés en ce que K représente un reste phényle ou naphtyle qui porte un ou plusieurs substituants pris panai sulfo, hydroxy, alkoxy en C₁-C₄, amino, N-(alkyl en C₁-C₄)- ou N,N-di-(alkyl en C₁-C₄)-amino, acétylamino, benzoylamino, alkyle en C₁-C₄, et phénylazo ou naphtylazo non substitué ou substitué par des substituants alkyle en C₁-C₄, alkoxy en C₁-C₄, halogène, sulfo, amino, N-(alkyl en C₁-C₄)- ou N,N-di-(alkyl en C₁-C₄)amino ou phénylamino ; un reste 1-phénylpyrazol-5-one ou 1-phényl-5-aminopyrazole non substitué ou substitué par des substituants alkyle en C₁-C₄, alkoxy en C₁-C₄, sulfo ou halogène ; un reste indole non substitué ou substitué par des substituants alkyle en C₁-C₄, alkoxy en C₁-C₄, sulfo ou halogène ; un reste naphtylimidazole non substitué ou substitué par des substituants alkyle en C₁-C₆, sulfo, hydroxy ou phénylamino, qui peut être substitué de son côté par des substituants alkyle en C₁-C₄, alkoxy en C₁-C₄, sulfo ou halogène.

4. Composés selon l'une des revendications 1 à 3, caractérisés en ce que K représente un reste 1- ou 2-naphtyle, qui porte un ou plusieurs substituants pris parmi hydroxy, amino, acétylamino, sulfo et chlore, ou représente un reste 1-phénylpyrazol-5-one ou 1-phényl-5-aminopyrazole chacun portant un ou plusieurs substituants pris dans le groupe comportant méthyle, méthoxy, sulfo et chlore.

5. Composés selon l'une des revendications 1 à 4, caractérisés en ce que R₁ et R₂ représentent chacun, indépendamment l'un de l'autre, un atome d'hydrogène, des groupes méthyle, éthyle, méthoxy, éthoxy, chlore ou sulfo.

6. Composés selon l'une des revendications 1 à 5, caractérisés en ce que (R₃)₀₋₃ représente 0 à 3 restes identiques ou différents pris dans le groupe comportant méthyle, éthyle, méthoxy, éthoxy, chlore ou sulfo.

7. Composés selon l'une des revendications 1 à 6, caractérisés en ce que X représente un élément de pontage de formule -SO₂-O-, -SO₂-N(alkyl en C₁-C₄)-, -COO- ou -CO-N(alkyl en C₁-C₄)-, et de préférence un groupe de formule -SO₂-O-.

8. Composés selon la revendication 1, caractérisés en ce que R₆ représente le reste -CH₂Cl, CHBr-CH₂Br ou -CBr=CH₂.

9. Composés selon l'une des revendications 1 à 8, caractérisés en ce qu'ils présentent un ou plusieurs groupes sulfo et de préférence un groupe sulfo.

10. Procédé pour la préparation de composés de formule (1) selon la revendication 1, caractérisés en ce qu'on copule une amine de formule 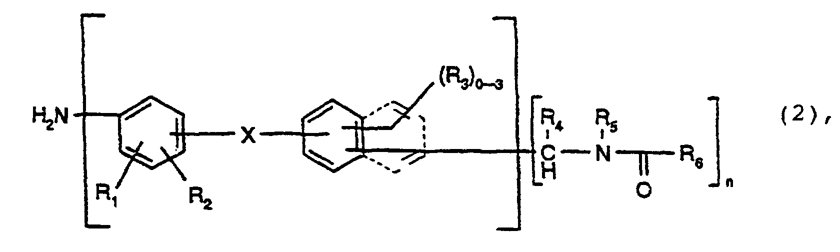 où R₁, R₂, (R₃)₀₋₃, R₄, R₅, R₆, R₇, X et n possèdent chacun la signification donnée auparavant, et on copule sur un composant de copulation de formule
K-H (3),
où K possède la signification donnée à la revendication 1.

11. Composés de formule (2) selon la revendication 10.

12. Utilisation de composés de formule (1) selon l'une des revendications 1 à 9 pour la teinture ou l'impression de matières fibreuses azotées ou contenant des groupes hydroxyle.

13. Utilisation selon la revendication 12 pour la teinture ou l'impression de matières fibreuses polyamides synthétiques, en particulier de la laine.
